# EUROPEAN PATENT APPLICATION

(11) **EP 1 262 189 A1**
(43) Date of publication of application: **04.12.2002**
(21) Application number: 01904528.5
(22) Date of filing: 19.02.2001
(51) Int. Cl.: A61K 38/22, A61P 9/12, A61P 15/08, A61P 3/04, A61P 3/08, A61P 9/10, A61P 5/02, A61P 37/02, A61P 43/00

(54) **LEPTIN-RESISTANCE AMELIORATING AGENTS**

(30) Priority: 06.03.2000 JP 2000060458
(71) Applicant: Sumitomo Pharmaceuticals Company, Limited, Osaka-shi, Osaka 541-8510 (JP)
(72) Inventor: NAKAGAWA, Tsutomu, Toyonaka-shi, Osaka 561-0802 (JP); YAMANAKA, Mitsugu, Nishinomiya-shi, Hyogo 662-0831 (JP); TAIJI, Mutsuo, Takatsuki-shi, Osaka 569-1044 (JP)
(74) Representative: Cresswell, Thomas Anthony
(86) International application number: JP0101149
(87) International publication number: WO01066133

(57) **Abstract**

Leptin resistance ameliorating agents comprising as the active ingredient a neurotrophic factor such as BDNF or a trk receptor ligand, namely, agents for ameliorating, treating or preventing hypertension, sterility, obesity, glucose metabolic disorders, ischemic diseases, growth hormone secretion insufficiency, growth hormone hyposecretion, immune function disorder, etc. caused by leptin resistance.

## Description

### TECHNICAL FIELD

The present invention relates to a leptin resistance ameliorating agent. More particularly, the present invention relates to a leptin resistance ameliorating agent comprising as the active ingredient a neurotrophic factor, namely, an agent which can improve leptin resistance and ameliorate, treat or prevent various diseases caused by the presence of leptin resistance by improving leptin resistance.

### BACKGROUND ART

Leptin is a peptide hormone produced by adipose cells, and has found as a factor acting in the hypothalamus of the brain to exhibit an inhibitory activity of food appetite and an activity of increasing energy expenditure. Therefore, leptin had been considered to exhibit anti-obesity activities, but it has been found that the serum leptin level of obese animals or obese patients is rather elevated in many cases. That is, it has been considered that there is a symptom named "leptin resistance" wherein although the serum leptin level of obese patients is high, that leptin does not exhibit its effects. In the developed countries, the number of obese patients has been increased due to a change of the life style, and the obesity becomes one of the social problems, because it can be a cause for lifestyle-related diseases such as hypertension, diabetes mellitus, or hyperlipemia.

In addition, new physiological activities of leptin have been found. Although ob/ob mice having an abnormality of leptin gene and exhibiting obesity have been known to lack a reproductive function, the administration of exogenous leptin makes them fertile, and it is suggested that a lack of leptin activity may possibly be a cause for sterility (Mounzih K. et al.: Endocrinology, 138:1190-1193, 1997). Further, leptin has been known to have angiogenesis activity (Sierra-Honigmann M.R. et al.: Science, 281:1683-1686, 1998), growth hormone release increasing activity (Carro E. et al.: Endocrinology 138:2203-2206, 1997), and activities on immune function (Ozata M. et al.: J of Clinical Endocrinology & Metabolism 84:3686-3695, 1999), and the lack of leptin activity may possibly be a cause for many diseases.

On the other hand, it has been reported that the presence of leptin resistance causes hyperleptinemia, by which disease states may be occurred secondary to the overaction of leptin. That is, when compared the serum leptin level of a person showing hypertension and a person having normal blood pressure but both persons being in a similar obese state, it was found that the serum leptin level of the patients with hypertension is higher (Agata J. et al.: Am. J. Hypertension, 10:1171-1174, 1997), and the transgenic mice with overexpression of leptin showed hypertension even though the animals were not obese (Ogawa Y. et al.: J. of Hypertension, 16 (suppl.2): S7, 1998), and hence, it has been suggested that hyperleptinemia may possibly be a cause for hypertension being independent on obesity.

The mechanism of forming leptin resistance has not been clarified yet, and there has not been known any medicament affecting on leptin resistance. Since a patient showing leptin resistance exhibits a high serum leptin level already, it can be speculated that the effects of leptin cannot be expected even though exogenous leptin is administered.

On the other hand, a neurotrophic factor is a generic name for proteins, which are provided from target cells or neurons and glial cells and Schwann cells in the living body, and show activities to maintain the survival and differentiation of neurons. They are classified into many types according to the kinds of nerves or receptors to function. Among them, proteins being known as neurotrophins have high structural homology with each other and form a family. The typical examples of neurotrophic factors are neurotrophins such as nerve growth factor (hereinafter, abbreviated as NGF), brain-derived neurotrophic factor (hereinafter, abbreviated as BDNF), neurotrophin 3 (hereinafter, abbreviated as NT-3), neurotrophin 4 (hereinafter, abbreviated as NT-4), neurotrophin 5 (hereinafter, abbreviated as NT-5), or neurotrophin 6 (hereinafter, abbreviated as NT-6); ciliary neurotrophic factor (hereinafter, abbreviated as CNTF); glial cell-derived neurotrophic factor (hereinafter, abbreviated as GDNF), etc. In addition, neurotrophins are known to act as a specific ligand of receptors including p75 and trk gene products (trkA, trkB and/or trkC) (cf. Takeshi NONOMURA, Hiroshi HATANAKA; Jikken Igaku, vol. 13, p. 376 (1995)).

### DISCLOSURE OF INVENTION

An object of the present invention is to provide a novel leptin resistance ameliorating agent, which can reduce leptin resistance and can ameliorate, treat or prevent obesity, hypertension, sterility, etc. caused by leptin resistance.

When the present inventors studied the pharmacological activities of BDNF, and administered BDNF to mice fed with high fat diet which had been known to exhibit leptin resistance (Surwit. R.S. et al.: Diabetes, 46: 1516-1520, 1997), they have found that the leptin resistance of the mice was improved by BDNF administration, and with further investigations, they have accomplished the present invention.

### BRIEF DESCRIPTION OF DRAWINGS

Fig. 1 is a graph showing the effects of BDNF on the diet consumption of the mice fed with high fat diet.
Fig. 2 is a graph showing the effects of BDNF on the body weight of the mice fed with high fat diet.
Fig. 3 is a graph showing the effects of BDNF on the serum leptin level of the mice fed with high fat diet.

### BEST MODE FOR CARRYING OUT THE INVENTION

That is, the present invention relates to the following:
(1) A leptin resistance ameliorating agent, which comprises as the active ingredient a neurotrophic factor;
(2) A leptin resistance ameliorating agent according to the above (1), wherein the neurotrophic factor is a brain-derived neurotrophic factor (BDNF);
(3) A leptin resistance ameliorating agent according to the above (1), wherein the neurotrophic factor is a nerve growth factor (NGF);
(4) A leptin resistance ameliorating agent according to the above (1), wherein the neurotrophic factor is neurotrophin 3 (NT-3);
(5) A leptin resistance ameliorating agent according to the above (1), wherein the neurotrophic factor is neurotrophin 4 (NT-4);
(6) A leptin resistance ameliorating agent according to the above (1), wherein the neurotrophic factor is neurotrophin 5 (NT-5);
(7) A leptin resistance ameliorating agent according to the above (1), wherein the neurotrophic factor is neurotrophin 6 (NT-6);
(8) A leptin resistance ameliorating agent according to the above (1), wherein the neurotrophic factor is ciliary neurotrophic factor (CNTF);
(9) A leptin resistance ameliorating agent according to the above (1), wherein the neurotrophic factor is glial cell-derived neurotrophic factor (GDNF);
(10) A leptin resistance ameliorating agent, which comprises as the active ingredient a ligand of a trkA, trkB and/or trkC receptor;
(11) A leptin resistance ameliorating agent, which comprises as the active ingredient a ligand of a trkB receptor;
(12) An agent according to one of the above (1) to (11), which can ameliorate, treat or prevent hypertension caused by leptin resistance;
(13) An agent according to one of the above (1) to (11), which can ameliorate, treat or prevent sterility caused by leptin resistance;
(14) An agent according to one of the above (1) to (11), which can ameliorate, treat or prevent the obesity caused by leptin resistance;
(15) An agent according to one of the above (1) to (11), which can ameliorate, treat or prevent glucose metabolic abnormality caused by leptin resistance;
(16) An agent according to one of the above (1) to (11), which can ameliorate, treat or prevent ischemic diseases caused by leptin resistance;
(17) An agent according to the above (16), wherein the ischemic disease is cerebral ischemia, ischemic heart disease or ischemia of lower extremities;
(18) An agent according to one of the above (1) to (11), which can ameliorate, treat or prevent growth hormone secretion insufficiency or growth hormone hyposecretion caused by leptin resistance;
(19) An agent according to one of the above (1) to (11), which can ameliorate, treat or prevent immune function disorders caused by leptin resistance; etc.

The present invention is illustrated in more detail below.

The "neurotrophic factor" means a physiologically active substance, which is secreted from the target cells for nerve growth, or by autocrine or paracrine, and promotes the growth, differentiation, or survival of neurons to form a neural circuit (synapse) in the living body. For example, the neurotrophic factor includes neurotrophins such as a nerve growth factor (hereinafter, abbreviated as NGF), a brain-derived neurotrophic factor (hereinafter, abbreviated as BDNF), a neurotrophin 3 (hereinafter, abbreviated as NT-3), a neurotrophin 4 (hereinafter, abbreviated as NT-4), a neurotrophin 5 (hereinafter, abbreviated as NT-5), and a neurotrophin 6 (hereinafter, abbreviated as NT-6); ciliary neurotrophic factor (hereinafter, abbreviated as CNTF); glial cell-derived neurotrophic factor (hereinafter, abbreviated as GDNF), etc. In addition, a modified recombinant neurotrophic factor produced by a substitution, a deletion, or an addition of a part of amino acid sequence of the naturally occurred neurotrophic factor sequence by a conventional technique may be included in the neurotrophic factor of the present specification, as far as it exhibits the similar physiological activities.

The "ligand of trkA, trkB and/or trkC receptor" is a generic name for substances that are bound to trkA, trkB or trkC, which is among the trk gene expression products being known as receptors for ''neurotrophin", and activate them to exhibit their activities. Concretely, the known neurotrophin includes, for example, NGF binding to trkA, BDNF and NT-4 binding to trkB, and NT-3 binding to trkC, etc. This concept includes not only modified neurotrophins (modified by amino acid substitution, deletion or addition or sugar chain modification), but also peptides and organic compounds of a lower molecular weight as far as they exhibit a binding ability and activating ability to trkA, trkB or trkC receptor, for example, ability of phosphorylating tyrosine residue.

Among the family of neurotrophic factors, BDNF, NT-3 and NT-4/5 exhibiting physiological activities thereof through trkB or trkC receptor, which is a trkB or trkC gene expression product, are more useful as a leptin resistance ameliorating agent. Among them, BDNF, which is known to exhibit its physiological activities through trkB receptor, i.e., a trkB gene expression product, is especially preferable as a leptin resistance ameliorating agent.

BDNF is a neurotrophic factor, that was isolated from porcine brain by Barde, Y.E. et al (cf. The EMBO Journal, vol. 5, p. 549-553 (1982)), and BDNF gene was analyzed after cloning, whereby it was confirmed that BDNF has a primary structure consisting of 119 amino acids (cf., Leibrock, J. et al., Nature, vol. 341, p. 149 (1989)). In addition, a modified recombinant BDNF such as Met-BDNF having a methionine residue at the N-terminus, or a recombinant mutant BDNF produced by a substitution, a deletion or a removal, or an addition of a part of amino acid sequence of the above naturally occurred BDNF sequence by a conventional gene engineering technique may be used in the present invention, as far as they are a pharmaceutical preparation having BDNF activities. The "BDNF activity" means activities of maintaining survival or promoting differentiation of a dorsal root ganglia, a ganglion inferius nervi vagi, a motoneuron, a retina ganglia, a nigradopaminergic neuron, a basal forebrain cholinergic neuron, etc. These activities can be confirmed *in vitro* or *in vivo* (JP-A-5-328974, USP 5,180,820).

In the present specification, the "leptin resistance" means a condition or symptom of the lack of leptin activity, which is occurred by generation of resistance to leptin, or reduction of sensitivity to leptin. It also includes reduction or failure of leptin activity in the signaling channel of leptin receptor or leptin of downstream of leptin receptor. In addition, the "leptin resistance" also includes overaction of leptin, which is occurred secondary to hyperleptinemia caused by leptin resistance. Further, leptin resistance may cause various diseases such as obesity, sterility, hypertension, glucose metabolic abnormality, ischemic diseases such as cerebral ischemia, ischemic heart disease or ischemia of lower extremities, growth hormone secretion insufficiency or growth hormone hyposecretion, etc.

In the present specification, the "leptin resistance ameliorating agent" means an agent exhibiting an activity of ameliorating the lack of leptin activity caused by leptin resistance. The "leptin resistance ameliorating agent" also includes an agent exhibiting an activity of ameliorating the reduction or failure of leptin activity in the signaling channel of leptin receptor or leptin at downstream of leptin receptor. Further, the "leptin resistance ameliorating agent" means an agent for ameliorating the overaction of leptin occurred secondary to hyperleptinemia caused by the presence of leptin resistance, more specifically, an agent for exhibiting an activity of ameliorating, treating or preventing disease states such as obesity, sterility, hypertension, glucose metabolic abnormality, ischemic diseases such as cerebral ischemia, ischemic heart disease or ischemia of lower extremities, growth hormone secretion insufficiency or growth hormone hyposecretion, and immune function disorder, etc.

In case of hyperleptinemia caused by the presence of leptin resistance, the "leptin resistance ameliorating agent" of the present invention may be administered to patients having a serum leptin level of not less than 15 ng/ml. Preferably, the present "leptin resistance ameliorating agent" may be administered to patients having a serum leptin level of not less than 20 ng/ml, more preferably to patients having a serum leptin level of not less than 25 ng/ml, and most preferably patients having a serum leptin level of not less than 30 ng/ml. The serum leptin level of healthy human is less than 10 ng/ml. (Methods for Preparation and Assay)

Any neurotrophic factor (NGF, BDNF, NT-3, NT-4, etc.) such as a naturally occurred one and a recombinant one, which is a leptin resistance ameliorating agent of the present invention, can be used in the present invention with purification, as far as it exhibits its inherent biological activities.

### (Preparation)

BDNF for the present leptin resistance ameliorating agent, for example, a naturally occurred one, a recombinant one, can be used in the present invention with purification, as far as it shows its inherent biological activities of BDNF. As a method for preparing BDNF, (1) extraction from BDNF producing tissue, (2) culture and isolation from BDNF producing cells (a primary culture cell or an established cell line), (3) culture and isolation from host cells being inserted with a recombinant DNA, etc., may be exemplified, but the method of (3) isolation from host cells being inserted with a recombinant DNA is usually suitable for production in a large scale.

Various methods for preparing these BDNF have been reported, and any BDNF, which is prepared by any method can be used in the present preparation. When a BDNF isolated from animal tissues is used in the present invention, it may be purified to such a degree that it can be used as a medicament (cf., The EMBO Journal, vol. 5, p. 549 (1982)). Alternatively, BDNF can be obtained from a primary culture cell or an established cell line that can produce BDNF, and isolating from the culture broth thereof (e.g., culture supernatant, cultured cells). Moreover, a recombinant BDNF can be obtained by a conventional gene engineering technique, e.g., by inserting a gene encoding BDNF into a suitable vector, transforming a suitable host with the recombinant vector, and isolating from a culture supernatant of the resulting - transformant (cf., Proc. Natl. Acad. Sci. USA, vol. 88, p. 961 (1991); Biochem. Biophys. Res. Commun., vol. 186, p. 1553 (1992)). The gene engineering technique is suitable for production of BDNF of same quality in a large scale. The host cells to be used in the above process is not limited, but may be any conventional host cells which have been used in a gene engineering technique, for example, *Escherichia coli*, *Bacillus subtilis*, yeasts, plant cells or animal cells.

### Method for preparation of NT-3:

NT-3 can be prepared by expressing in various host cells in the same manner as in the preparation of BDNF. The methods for preparing thereof and the methods for assay thereof are disclosed in Neuron, vol. 4, 767-773 (1990), or JP-A-5-161493 (WO 91/3659).

### Method for Preparation of Nt-4:

NT-4 can be prepared by expressing in various host cells in the same manner as in the preparation of BDNF. The methods for expression of the recombinant NT-4 and the methods for assay thereof are disclosed in Proc. Natl. Acad. Sci. USA, vol. 89, p. 3060-3064 (1992.4), JP-A-7-509600 (WO 93/25684), or JP-A-6-501617 (WO 92/5254).

### Method for Preparation of CNTF:

CNTF can be prepared in a large scale by expressing in various host cells in the same manner as in the preparation of BDNF. The methods for expression of the recombinant CNTF and the methods for assay thereof are disclosed in Biochimica et Biophysica Acta, vol. 1090, p. 70-80 (1991), J. Neurochemistry, vol. 57, p. 1003-1012 (1991). The methods for expressing the recombinant CNTF and the assay thereof are disclosed in JP-A-4-502916 (WO 90/7341).

The leptin resistance ameliorating agent of the present invention, which comprise as the active ingredient a neurotrophic factor, can be administered either parenterally or orally.

The precise dosage and the administration schedule of the leptin resistance ameliorating agent of the present invention should vary according to the method for treatment to be required for each patient, the disease to be treated, or the degree of necessity, and further according to the diagnosis by a physician. When administered parenterally, the dosage and the frequency of the administration may vary according to the conditions, ages, body weights of patients, and administration routes, but when it is administered subcutaneously or intravenously in the form of an injection, then the daily dosage thereof is in the range of about 1 to about 2500 µg, preferably in the range of about 10 to about 500 µg, more preferably in the range of about 20 to about 200 µg, per 1 kg of the body weight in an adult. When it is administered to the air tract in the form of an aerosol spray, the daily dosage thereof is in the range of about 1 µg to about 2500 µg, preferably in the range of about 10 to about 500 µg, more preferably in the range of about 20 to about 200 µg, per 1 kg of the body weight in an adult. The administration schedule is either continuous daily administration, intermittent administration, or a schedule of combining these methods. When administered orally, the dosage and the frequency of administration may vary according to the conditions, ages, body weights of patients, and administration routes, and the daily dosage thereof is in the range of about 5 to about 2500 µg, preferably in the range of about 10 to about 1000 µg per 1 kg of the body weight in an adult.

A pharmaceutical composition can be prepared by mixing a neurotrophic factor with a pharmaceutically acceptable non-toxic carrier. When a pharmaceutical composition for parenteral administration (subcutaneous injection, intramuscular injection, or intravenous injection) is prepared, it is preferably in the form of a solution preparation or a suspension preparation. When a pharmaceutical composition for intravaginal administration or rectal administration is prepared, it is preferably in the form of a semi-solid preparation such as cream or suppository. When a pharmaceutical composition for intranasal administration is prepared, it is preferably in the form of a powder, a nasal drop, or an aerosol.

The pharmaceutical composition is administered in the form of a single dosage unit, and can be prepared by any conventional method that is known in the pharmaceutical field such as methods disclosed in Remington's Pharmaceutical Science (published by Mack Publishing Company, Easton, PA, 1970). An injection preparation may optionally contain as a pharmaceutical carrier a protein derived from plasma such as albumin, an amino acid such as glycine, or a carbohydrate such as mannitol, and additionally a buffering agent, a solubilizer, or an isotonic agent, etc. can be contained. When the present pharmaceutical composition is in the form of an aqueous solution preparation or a lyophilized preparation, it may preferably contain a surfactant such as Tween 80 (registered trade mark), Tween 20 (registered trade mark), etc. in order to avoid aggregation. When the present pharmaceutical composition is a composition for parenteral administration other than an injection preparation, then it may contain distilled water or physiological solution, polyalkylene glycol such as polyethylene glycol, an oil derived from plant, hydrogenated naphthalene, etc. For example, a pharmaceutical composition such as a suppository for intravaginal administration or rectal administration may contain as a conventional excipient polyalkylene glycol, vaseline, cacao butter, etc. A pharmaceutical composition for intravaginal administration may contain an absorbefacient such as a bile salt, an ethylenediamine salt, a citrate, etc. A pharmaceutical composition for inhalation may be in the form of a solid preparation, and may contain as an excipient lactose, etc., and a pharmaceutical composition for intranasal drop may be in the form of an aqueous solution or an oily solution.

The present pharmaceutical composition is especially preferable in the form of a formulation by which the present compound can persistently be given to a subject by a single administration for a long term, e.g., for one week to one year, and various sustained release preparations, depot preparations, or implant preparations can be employed. For example, a pharmaceutical composition may contain a neurotrophic factor per se, or a pharmaceutically acceptable salt of a neurotrophic factor having a low solubility in body fluid. Such pharmaceutically acceptable salts are, for example, (1): an acid addition salt such as phosphate, sulfate, citrate, tartrate, tannate, pamoate, alginate, polyglutarate, naphthalenemono- or di-sulfonate, poly-galacturonate, etc., (2): a salt or complex with polyvalent metal cation such as zinc, calcium, bismuth, barium, nickel, etc, or a combination of (1) and (2), for example, a tannic acid-zinc salt, etc. A neurotrophic factor is preferably converted into a slightly-water-soluble salt thereof, which is mixed with a gel, for example, aluminum monostearate gel and sesame oil, etc. to give a suitable injection preparation. In this case, especially preferable salt is a zinc salt, a tannic acid-zinc salt, a pamoate, etc. Another type of a sustained release injection preparation is ones wherein a neurotrophic factor is preferably converted into a slightly-water-soluble salt thereof, which is further enclosed in a slow-disintegrative non-toxic and non-antigenic polymer such as a polymer or a copolymer of polylactic acid/polyglycolic acid. In this case, especially preferable salt is zinc salt, tannic acid-zinc salt, pamoate, etc. In addition, a neurotrophic factor or a slightly-water-soluble salt thereof can be enclosed into a cholesterol matrix or a collagen matrix to give a sustained release preparation.

The pharmaceutical preparation for oral administration may be ones which are prepared by microencapsulating a neurotrophic factor or a salt thereof with lecithin, cholesterol, a free fatty acid, or ones which are prepared by enclosing said microcapsules into gelatin capsules, or ones which are prepared by enclosing a neurotrophic factor or a salt thereof in enteric capsules, etc. These preparations may additionally contain, for example, an absorbefacient, a stabilizer, a surfactant, etc.

### (Toxicity)

When a neurotrophin, especially BDNF, was administered subcutaneously to rats and cynomolgus monkeys at a dose of 100 mg/kg and 60 mg/kg, respectively, for four weeks, no animal died. With respect to the acute toxicity, BDNF was administered to rats and cynomolgus monkeys at a dose of 200 mg/kg or more, and no animal died. Therefore, BDNF shows high safety.

### EXAMPLES

The present invention is illustrated by Examples.

### Example 1

### Effects of BDNF on the mice fed with high fat diet:

### (1) Materials for experiments:

### Reagents:

BDNF was purchased from REGENERON PHARMACEUTICALS, INC., and used. The other reagents were commercially available ones with the best quality.

### Test animals:

Male C57 BL/6N mice (SPF) were purchased from Charles River Japan Inc. After pre-feeding, the animals were used in the experiment at 8 weeks old.

### Breeding conditions:

The mice were kept in a room being controlled at a temperature of from 20°C to 26°C under a relative humidity of from 30 % to 70 %, with an illumination cycle of light on (8:00 to 20:00) and light off (20:00 to 8:00). During the pre-feeding, the animals were given diet (CE-2, Clea Japan, Inc.) and sterilized tap water *ad libitum*.

### (2) Production of mice fed with high fat diet:

C57 BL/6N mice were grouped into 2 groups. Regular diet was given to one of the groups, and a high fat diet (CE-2 + 30 % lard) was given to the remaining group, and the mice were kept for 2 months.

### (3) Preparation of test solution:

As a BDNF solution, a test compound solution (10 mM phosphoric acid, 150 mM sodium chloride, 0.01 % Tween 80, 1 % mannitol) containing BDNF at a final concentration of 20 mg/ml was prepared. Separately, a vehicle control solution containing no BDNF (10 mM phosphoric acid, 150 mM sodium chloride, 0.01 % Tween 80, 1 % mannitol) was prepared.

### (4) Administration of test compound:

BDNF was administered subcutaneously at a dose of 20 mg/kg (1 ml/kg), and to the control group, the vehicle was administered subcutaneously at a dose of 1 ml/kg. The administration was carried out once a day for 6 days.

### (5) Effects of BDNF on the food consumption and the body weight of the mice fed with the high fat diet:

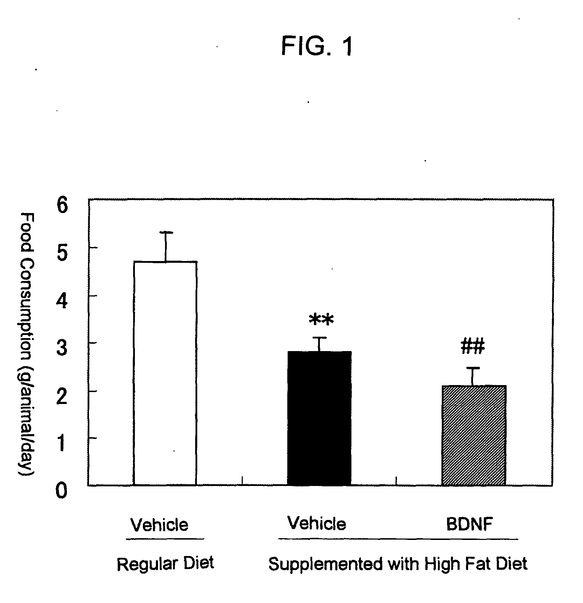

To the mice fed with a high fat diet for 2 months as mentioned above, BDNF or the vehicle was administered for 6 days, and then, the weight of the food box was measured, and the amount of the food consumption was calculated by subtracting the amount of the remained food from the total amount of the food to be given. The effects of BDNF on the food consumption of the mice are shown in Fig. 1.

In Fig. 1, the white column indicates the average food consumption over the administration period of the mice that were fed with regular diet and the vehicle, the black column indicates the average food consumption over the administration period of the mice feed with the high fat diet and the vehicle, and the shaded black column indicates the average food consumption over the administration period of the mice fed with the high fat diet and BDNF. The data are expressed in mean value ± SD of each group (n=7). ** indicates that there is a significant difference of p<0.01 to the group fed with the regular diet and the vehicle by Student's t-test. ## indicates that there is a significant difference of p<0.01 to the group fed with the high fat diet and the vehicle by Turkey's test.

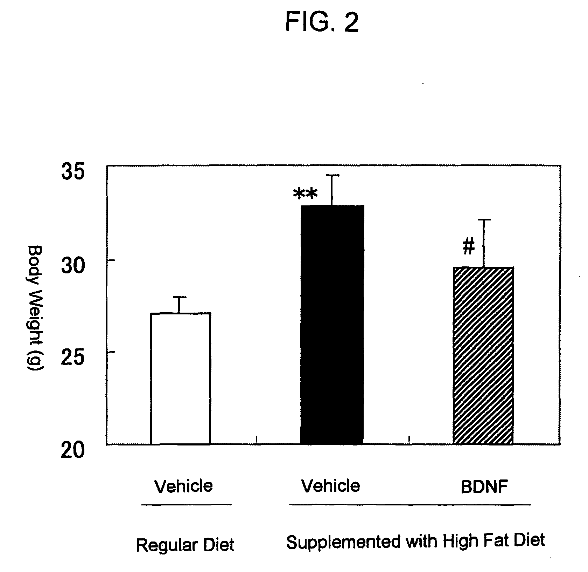

The body weights of the mice fed with BDNF or the vehicle for 6 days as mentioned above were measured with a scale for animal. The effects of BDNF on the body weight of the mice fed with the high fat diet are shown in Fig. 2.

In Fig. 2, the white column indicates the body weight on the 6th day of the mice fed with the regular diet and the vehicle, the black column indicates the body weight on the 6th day of the mice fed with the high fat diet and the vehicle, and the shaded black column indicates the body weight on the 6th day of the mice fed with the high fat diet and BDNF. The data are expressed in mean value ± SD of each group (n=7). ** indicates that there is a significant difference of p<0.01 to the group fed with the regular diet and the vehicle by Student's t-test. # indicates that there is a significant difference of p<0.05 to the group fed with the high fat diet and the vehicle by Turkey's test.

As is apparent from the graph of Fig. 1, it was found that BDNF reduced the food consumption of the mice fed with the high fat diet. In addition, as is shown in Fig. 2, the mice fed with the high fat diet got fatter than the mice fed with the regular diet, but by administration of BDNF, the obesity of the mice fed with the high fat diet was improved.

### (6) Effects of BDNF on glucose tolerance of the mice fed with the high fat diet:

To the mice fed with the high fat diet for 2 months as mentioned above, BDNF or the vehicle was administered for 6 days, and then the mice were fasted overnight. Glucose was orally administered to the mice in an amount of 3 g/kg of the body weight, and the mice were subjected to glucose loading for 90 minutes. The blood glucose level of the mice was measured as mentioned below, and the effects of BDNF on the glucose tolerance was studied.

Namely, the blood (10 µl) was taken from the tail vein of the mice, and mixed with 0.4 N aqueous perchloric acid solution (100 µl). The mixture was neutralized with 0.37 M aqueous potassium carbonate solution (50 µl), and centrifuged. The glucose concentration in the supernatant was measured with Glucose CII Test Wako (a kit for assay of glucose, mutarotase GOD method, manufactured by Wako Pure Chemical Industries, Ltd.). The results are shown in Table 1.

**Table 1:**

| Evaluation by oral glucose tolerance test | | |
|---|---|---|
| Regular Diet | High Fat Diet | |
| Vehicle | Vehicle | BDNF |
| 159.6 ± 15.4 | 189.4 ± 24.6** | 154.9 ± 10.7^{##} |

The data in Table 1 indicate the blood glucose level (mg/dl) on 90 minutes after the glucose administration, and are expressed in mean value ± SD of each group (n=7). ** indicates that there is a significant difference of p<0.01 to the group fed with the regular diet and the vehicle by Student's t-test. ## indicates that there is a significant difference of p<0.01 to the group fed with the high fat diet and the vehicle by Turkey's test.

As is shown in the results of the above Table 1, it was found that the blood glucose level on 90 minutes after the glucose administration of the mice fed with the high fat diet was significantly higher than that of the mice fed with the regular diet, and the mice fed with the high fat diet showed impaired glucose tolerance. Further, it was found that the blood glucose level was significantly reduced by BDNF administration, and that BDNF exhibits an activity of ameliorating the impaired glucose tolerance of the mice fed with the high fat diet.

### (7) Effects of BDNF on serum leptin level:

To the mice fed with the high fat diet for 2 months as mentioned above, BDNF or the vehicle was administered for 6 days, and the blood was taken from the tail vein of the mice on the 6th day, and then the serum was separated therefrom. The serum leptin level was measured with a commercially available mouse leptin ELISA kit (AN'ALYZA mouse Leptin, manufactured by Genzyme Techne, Ltd.).

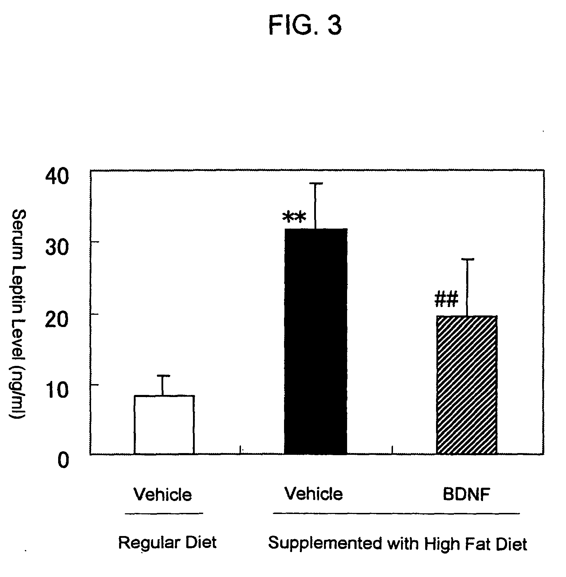

The results are shown in Fig. 3. In the figure, the data show those on the 6th day of the administration, wherein the white column indicates the data of the mice fed with the regular diet and the vehicle, the black column indicates the data of the mice fed with the high fat diet and the vehicle, and the shaded black column indicates the data of the mice fed with the high fat diet and BDNF. The data are expressed in mean value ± SD of each group (n=7). ** indicates that there is a significant difference of p<0.01 to the group fed with the regular diet and the vehicle by Student's t-test. ## indicates that there is a significant difference of p<0.01 to the group fed with the high fat diet and the vehicle by Turkey's test.

As is shown in Fig. 3, it was found that the serum leptin level of the mice fed with the high fat diet was significantly higher than that of the mice fed with the regular diet, and the mice fed with the high fat diet showed leptin resistance, and further it was found that the serum leptin level was significantly reduced by BDNF administration and BDNF exhibited an activity of ameliorating leptin resistance.

### INDUSTRIAL APPLICABILITY

The leptin resistance ameliorating agent of the present invention improves the lack of leptin activity caused by leptin resistance, and further exhibits activities of ameliorating, treating or preventing obesity or sterility. In addition, the present agent ameliorates the overaction of leptin occurred secondary to hyperleptinemia caused by the presence of leptin resistance, and exhibits activities of ameliorating, treating or preventing disease states such as hypertension, etc.

## Claims

1. A leptin resistance ameliorating agent, which comprises as the active ingredient a neurotrophic factor.

2. A leptin resistance ameliorating agent according to claim 1, wherein the neurotrophic factor is a brain-derived neurotrophic factor.

3. An agent according to any one of claim 1 and claim 2, which ameliorates, treats or prevents hypertension caused by leptin resistance.

4. An agent according to any one of claim 1 and claim 2, which ameliorates, treats or prevents sterility caused by leptin resistance.

5. An agent according to any one of claim 1 and claim 2, which ameliorates, treats or prevents obesity caused by leptin resistance.

6. An agent according to any one of claim 1 and claim 2, which ameliorates, treats or prevents glucose metabolic abnormality caused by leptin resistance.

7. An agent according to any one of claim 1 and claim 2, which ameliorates, treats or prevents ischemic disease caused by leptin resistance.

8. An agent according to any one of claim 1 and claim 2, which ameliorates, treats or prevents growth hormone secretion insufficiency or growth hormone hyposecretion caused by leptin resistance.

9. An agent according to any one of claim 1 and claim 2, which ameliorates, treats or prevents immune function disorders caused by leptin resistance.

10. A leptin resistance ameliorating agent, which comprises as the active ingredient a ligand of a trkA, trkB or trkC receptor.
